# EUROPEAN PATENT APPLICATION

(11) **EP 2 168 615 A1**
(43) Date of publication of application: **31.03.2010**
(21) Application number: 09171319.8
(22) Date of filing: 25.09.2009
(51) Int. Cl.: A61M 3/02

(54) **Device for rectal lavage**

(30) Priority: 26.09.2008 IT PR20080058
(71) Applicant: Franceschi, Claude, 92100 Boulogne (FR); De Cillia, Giancarlo, 43100 Pharma (IT)
(72) Inventor: Franceschi, Claude, 92100 Boulogne (FR); De Cillia, Giancarlo, 43100 Pharma (IT)
(74) Representative: Conti, Marco

(57) **Abstract**

A device (1) for rectal lavage comprises a tubular body (2), forming a passage (3) having an inlet (4) connectable to a source of pressurised liquid and an outlet (5) consisting of an opening formed in a lateral wall of the tubular body (2), a plug (7) operatively fitted into the passage (3) in a plugging station (8) and axially movable between positions for opening and closing the passage (3), and means of actuating the device operatively active on the plug (7) to generate a jet of liquid discharged from said opening and direct it towards the anus for lavage of the rectum; said actuating means consist of a manoeuvring member (9) slidably coupled to the tubular body (2) so as to be movable along the axis of the plug itself (7), the manoeuvring member (9) being rigidly connected to the plug (7) in order to move it directly.

The known devices for washing the rectum in a non-invasive manner do not allow the person who uses them simple and effective control of the delivery of the jet, and for this reason they are inconvenient to use.

## Description

The subject of the present invention is a device for rectal lavage.

The technical field of the invention is that of devices for hygiene and cleaning the body, and in particular for lavage of the external and internal anal area.

In this field, a device is known (from patent document EP0462871 by the same Applicant) for rectal hygiene which enables a jet of water to be generated and directed towards the anus while maintaining the device at a certain distance from the body (without therefore inserting the device into the rectum).

This instrument is consequently not invasive, because it does not come into contact with the person. It is therefore a non-sterile, reusable instrument.

If the jet of water is suitably shaped, the jet of water is capable of washing the external anal surface (without therefore entering the rectum) or the internal anal surface (therefore entering the rectum), according to the position in which the device is maintained with respect to the anal orifice.

It is therefore very important for the device to be easy to handle and to allow precise and effective control of the intensity and position of the jet.

The known device comprises a tube forming a passage, having an inlet connectable to a source of pressurised liquid and an outlet consisting of an opening formed in a lateral wall of the tube, at an opposite end with respect to the water inlet.

The device is provided with a valve for plugging the passage. This valve comprises a plug fitted into the passage in a plugging station and axially movable therein between positions for opening and closing the passage.

To actuate the valve, thus activating or deactivating the jet of water, the device provides for acting on a rod connected to the plug, twisting it with the hands or inclining it through the action of a button located perpendicularly to the rod. In this way, the plug is displaced from its seat by means of the inclination of the plug, bringing about the opening of the valve.

However, this device has the disadvantage of not allowing adequate control of the delivery of the fluid, which is therefore generated in an excessively rapid and disruptive manner.

This prejudices the ease and practicality of use of the device, which (especially for someone who does not have experience of its use) risks being really annoying, for example because the jet generated is too vigorous or because its direction is not well controlled, and for this reason the person risks wetting themselves in an undesirable fashion.

These disadvantages are particularly serious if we consider that the device is designed for everyday use, for intimate daily cleaning; for this reason it is important that the means of actuating the device should be practical to use and should ensure a gradual and controlled delivery of the jet.

It is an object of the present invention to eliminate the aforementioned drawbacks and to make available a device for rectal lavage actuatable simply with one hand, resulting in a gradual and controlled delivery of the jet of water.

Said object is fully achieved by the device which is the subject of the present invention, characterised by what is contained in the claims set forth below and in particular by the fact that said actuating means comprise a manoeuvring member slidably coupled to the tubular body so as to be movable along the axis of the plug itself, the manoeuvring member being connected to the plug in order to move it.

This and other characteristics will become clearer from the following description of a preferred embodiment, illustrated, purely by way of non-limiting example, in the attached drawings, in which:
- figure 1 illustrates a device according to the present invention;
- figure 2 illustrates the device shown in figure 1, in an exploded and sectioned view;

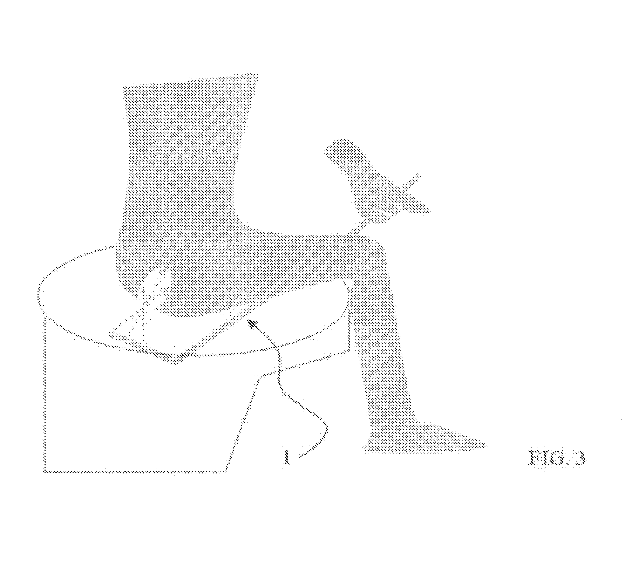

- figure 3 schematically illustrates the device shown in figure 1, during its use by a person;
- figure 4 illustrates a portion of the device shown in figure 1, in section;
- figure 5 illustrates an opening in the device shown in figure 1, according to a first variant embodiment;
- figure 6 illustrates the opening shown in figure 5, according to a second variant embodiment;
- figure 7 illustrates the device shown in figure 1, in an exploded and sectioned view, according to a variant embodiment.

In the figures, no. 1 indicates a device for rectal lavage, according to the present invention.

The device 1 comprises a tubular body 2 forming a passage 3 having an inlet 4 connectable to a source of pressurised liquid (preferably water) and an outlet 5 consisting of an opening 15 (or a slit) formed in a lateral wall of the tubular body 2.

In proximity to the inlet 4, the tubular body 2 is shaped so as to form a grip 6, to allow a person to hold the device 1 firmly in the hand.

Tubular body 2 is shaped so as to form at least a first portion 2A (in which the grip 6 is also present) and a second portion 2B which is bent relative to the first at a determinate angle.

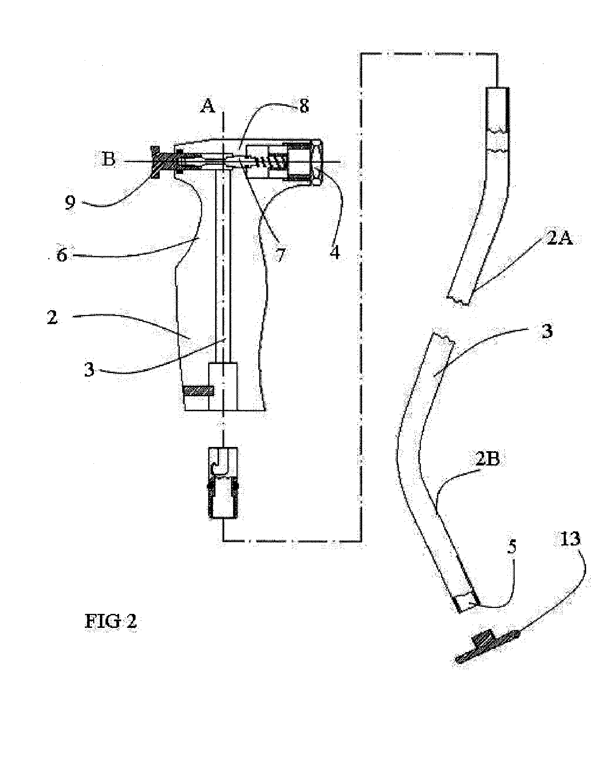

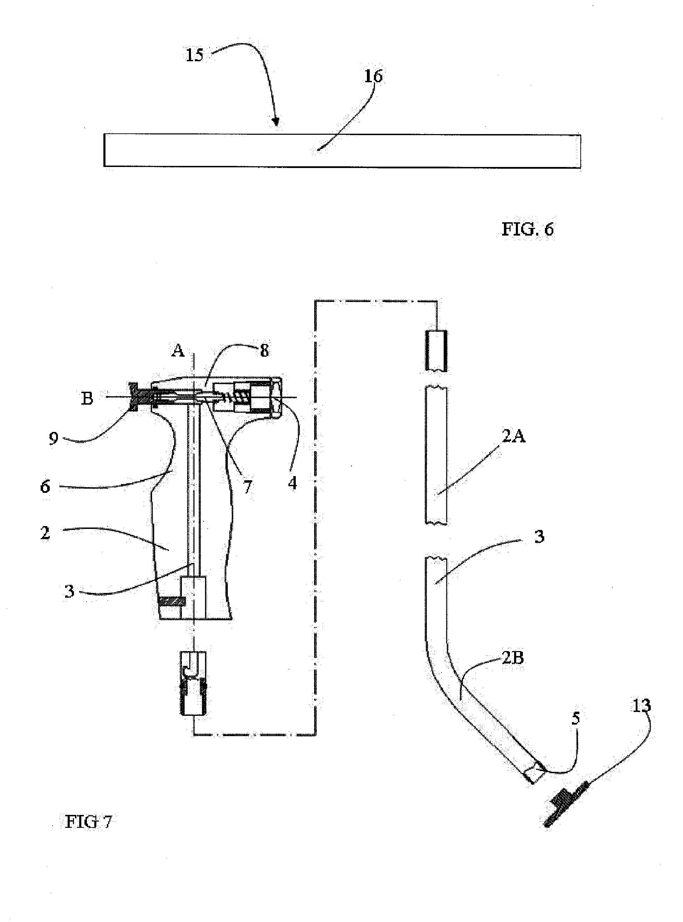

In this way, the passage 3 has a deviation equal to this determinate angle, at its end, or in proximity to the outlet 5. The angle formed by the portions 2A and 2B of the tubular body 2 is preferably of about 40 degrees. This facilitates use by a person in a sitting position (in this connection see figure 2 or figure 7). Preferably, the second portion 2B of the tubular body 2 is substantially rectilinear.

As regards the first portion 2A, it is preferably bent so that the tubular body 2 substantially forms an "S", as illustrated in figure 2. It should be noted however that the tubular body 2 has an extension substantially along a plane. Therefore, the portion of tubular body 2 forming the grip 6 and the second portion 2B of the tubular body 2 are located in opposed half-planes with respect to the direction along which lies the first portion 2A of the tubular body 2 subsequent to the grip. Therefore the tubular body 2 is preferably shaped so as to form a first portion 2A and a second portion 2B, which is bent relative to the first at a determinate angle, the first portion 2A forming preferably a first section curved in a direction opposite to that of the second portion 2B.

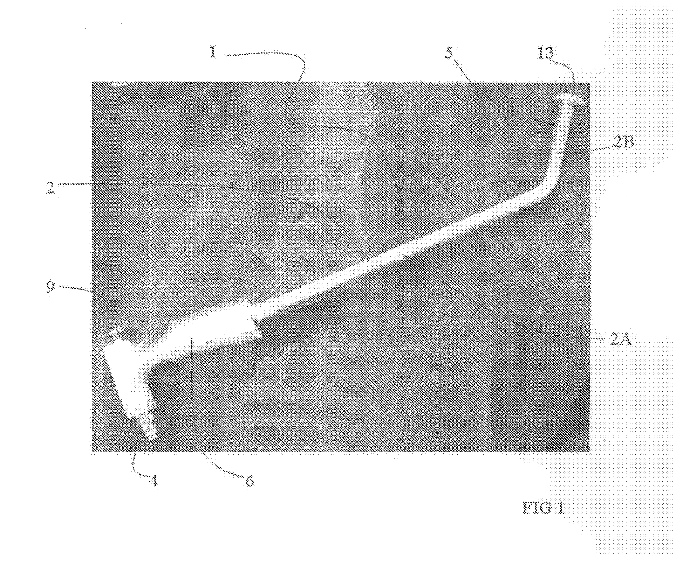

Again as regards the first portion 2A of the tubular body 2, it is envisaged according to a different embodiment (illustrated in figures 1 and 7) that it could be rectilinear.

In any case, it should be noted that the tubular body 2 extends substantially along a longitudinal path A, forming two or three (depending on the embodiment) rectilinear sections connected together and located in a plane.

In particular, in the embodiment illustrated in figure 2, the portion of the tubular body 2 forming the handle 6, and the second portion 2B of the tubular body 2, are bent to opposite sides with respect to the first portion 2A of the tubular body 2 (connected to the second portion 2B of the tubular body 2).

In the embodiment illustrated in figures 1 and 7, the second portion 2B of the tubular body 2 is bent with respect to the first portion 2A of the tubular body 2 (connected to the second portion 2B of the tubular body 2), the portion of tubular body 2 forming the handle 6 being aligned (not bent) with respect to the remaining part of the first portion 2A of tubular body 2.

The fact that the end portion 2B of the tubular body is bent with respect to said longitudinal direction has the function of allowing the person, by grasping the device 1 at the grip 6, to hold the device between the legs in a sitting position, while keeping the outlet 5 facing the anal orifice (at a certain distance from the latter, for example several centimetres).

The embodiment illustrated in figure 2 is particularly convenient also for people who have a protruding (or swollen) stomach or belly; in fact, for such people the embodiment of the device illustrated in figures 1 and 7 could have the drawback of having to hold the handle 6 too close to the belly during the use of the device (and for this reason the device itself would risk being uncomfortable).

The apparatus 1 also comprises a plug 7 operatively fitted into the passage 3 in a plugging station 8 and axially movable therein (i.e. along the axis of the section of the passage 3 into which it is fitted) between positions for opening and closing the passage 3.

The plug 7 constitutes a plugging valve, in other words a valve for opening and closing the passage 3.

It is important to note that upstream of this plugging valve (in other words above the plug 7), i.e. between the inlet 4 and the plug 7, the liquid is under pressure.

The apparatus 1 comprises a manoeuvring member 9 slidably coupled to the tubular body 2 so as to be movable along the axis of the plug 7 itself; the manoeuvring member is connected to the plug 7 to move it directly, thus forming means of actuating the device 1 which are operatively active on the plug 7 to generate a jet of liquid discharged from said opening and direct it towards the anus for washing the rectum.

Preferably, the manoeuvring member is rigidly connected to the plug 7 to move it directly, thus optimising the response of the plug 7 to action on the manoeuvring member 9.

The manoeuvring member 9 is substantially a pusher connected to the plug 7 to move it in the direction of opening the passage 3, overcoming the pressure of the liquid upstream of the valve.

The device 1 also comprises, preferably, returning means (for example an opposing spring 10) operatively active on the plug 7 to maintain it in the closing position, in the absence of action on the manoeuvring member 9.

It should be observed that the grip 6 is originally shaped so as to allow it to actuate said manoeuvring member 9 with the thumb of the hand holding the device 1, without the need to use two hands.

In the preferred embodiment illustrated, the tubular body 2 has, in proximity to the handle 6, a section of passage 3 forming the inlet for the liquid 4 and the plugging station 8, located in a substantially radial direction with respect to the longitudinal axis A formed by portion 2A of the tubular body; in this way, the plug 7 and the manoeuvring member 9 are movable along said radial direction.

The liquid therefore enters the tubular body 2 radially and flows through an initial section of the passage 3 (formed inside the handle 6) which is substantially transverse with respect to the longitudinal axis A of the tubular body 2.

Therefore the plug 7 (in other words the plugging valve) is operatively fitted in a section of the passage 3 which is substantially transverse (i.e. substantially perpendicular) with respect to the longitudinal axis A of the tubular body 2.

The passage 3 therefore extends along at least three sections; starting from the inlet 4 for the liquid, an initial section of the passage 3 extends along an axis B located substantially radially with respect to the axis of the tubular body 2; an intermediate section (which preferably constitutes about 80% of the length of the passage 3) located along the longitudinal axis A of the tubular body 2, and more precisely of the first portion 2A of the tubular body 2 (in the embodiment illustrated in figures 1 and 7, said section is rectilinear, while in the embodiment illustrated in figure 2, said section has a curvature), and a final section located along the axis of the second portion 2B of the tubular body 2, which is inclined by said determinate angle with respect to the axis A of the first portion 2A of the tubular body 2 which is connected to it.

As has been said earlier, these axes preferably lie in a single plane; in other words the passage 3 extends along a plane.

Furthermore, the second bent portion 2B of the tubular body 2 is preferably bent in the opposite direction to that in which the initial section of the passage 3 extends; in other words, the inlet 4 and the outlet 5 of passage 3 are located on opposite sides with respect to the longitudinal axis A, in a manner which is overall particularly comfortable and effective.

This allows a person to hold the device 1 firmly in one hand, grasping it by the grip 6, and comfortably actuate the manoeuvring member 9 by pressing it with the thumb of the same hand which grips the device 1, while maintaining the liquid outlet 5 correctly in the desired position (facing the anal orifice, from a seated position).

The fact that the plug 7 is located transversely to the longitudinal axis A of the tubular body 2 advantageously allows the plug to be actuated directly by operating the manoeuvring member 9 from a particularly comfortable position (as described above), without the need to twist or incline rods or act on the plug 7 indirectly by means of levers. Furthermore, the plug 7 is axially displaced so as to give precise control over its insertion (greater or lesser) in the plugging station 8, thus allowing adjustment, or rather metering, of the flow rate of liquid through passage 3. This result is possible as a result of the fact that the plug and the manoeuvring member are movable along the same axis (in other words, axis B), and that they are connected together in a substantially rigid manner, in other words in such a way that a determinate axial displacement of the manoeuvring member 9 is matched by a determinate axial displacement of the plug 7.

In the preferred embodiment illustrated, the actuating means comprise a rod-shaped body 11 movably coupled to a seat formed by the tubular body 2 and having a first portion projecting from the tubular body 2 to form said manoeuvring member 9 (in the form of a button) and a second portion operatively active on passage 3 in the plugging station to form the plug 7.

In other words, the button which constitutes the manoeuvring member 9 and the plug 7 are rigidly connected to each other to form a single rod-shaped body 11.

It should be noted that the plugging station 8 forms a constriction in the passage 3 in which the plug 7 is designed to fit in order to close off the passage.

A seal ring 12 is applied to the plug 7 to close the passage 3, so as to form a seal by pressing against a radial wall of the plugging station 8 formed by said constriction, when the plug 7 is in the closing position.

When the button is pressed, in other words the manoeuvring member 9, the seal ring 12 moves axially (along axis B), toward the entrance 4 of the tube, thus moving away from the wall of the plugging station 8, allowing the liquid to flow along the passage 3.

However, even when the seal ring 12 is moved away from the closing position, the plug 7 partially blocks the passage 3 (in the area of the constriction to passage 3 formed by the plugging station 8).

As the manoeuvring member 9 is actuated (in other words as the button is pressed), the plug 7 (rigidly connected to the manoeuvring member 9) moves away from, in other words is withdrawn from, the plugging station 8, progressively freeing the passage 3 and therefore allowing a progressively greater flow of liquid.

This advantageously allows the person directly and effectively to control the flow of liquid and therefore the jet generated by the device 1.

In the light of this, considerable importance is attached to the shape of the plug 7, which determines the variation in the flow of liquid, corresponding to a determinate displacement of the plug 7, in other words of the manoeuvring member 9.

In the preferred embodiment illustrated, the plug 7 has a variable diameter that is monotone non-decreasing in the direction of insertion of the plug (7) in the constriction (i.e. in the plugging station 8).

In particular, the plug 7 comprises a first portion with a diameter increasing up to a determinate value, and a second cylindrical portion with a diameter of said determinate value, subsequent to the first portion in the direction of insertion of the plug 7 in the constriction to the plugging station 8.

More precisely, starting from the end-of-stroke abutment of the plug 7 (in other words from the seal ring 12), the profile of the plug 7 presents a cylindrical part (having for example a length of about 2 mm) which effectively limits spraying during the first part of the travel of the plug 7 itself, i.e. when the pressure of the liquid on the plug 7 is at its maximum, and the opening movement effected by the person's thumb is most difficult to control; the profile of the plug 7 is then reduced, with a taper section (preferably with an angle of 8 degrees and a length for example of 8 mm), and subsequently with a further taper section with a larger angle (preferably 20 degrees, for example for a length of 3 mm).

Said preferred conformation of the plug 7 (depending on the conformation of the plugging station 8 into which it fits) ensures a particular progressiveness of the jet, requiring minimum effort on the part of the person actuating the device 1.

As regards the outlet 5 of the passage 3, constituted by the opening 15 formed in the lateral wall of the tubular body 2 (as described above), it should be noted that its conformation is important as regards the characteristics of the jet generated.

In fact the jet must be conformed so as to generate a crossing at a determinate distance from the opening 15, in other words a zone in which the jet narrows, and then widens out.

To this end, opening 15 preferably has an elongated shape.

In the preferred embodiment illustrated, the opening 15 is formed in the second portion 2B of the tubular body 2, located along a determinate axis.

Opening 15 (in particular the cross-section of opening 15) has an extension of about 14 square mm; furthermore, opening 15 preferably forms a slit elongated in the direction of said axis (i.e. in the direction defined by section 2B of the tubular body in which it is situated).

In particular, a first and a second embodiment are provided for the opening; both produce a jet of laminar shape forming a crossing; the two embodiments differ as regards the distance at which the jet forms the crossing, with respect to opening 15 itself.

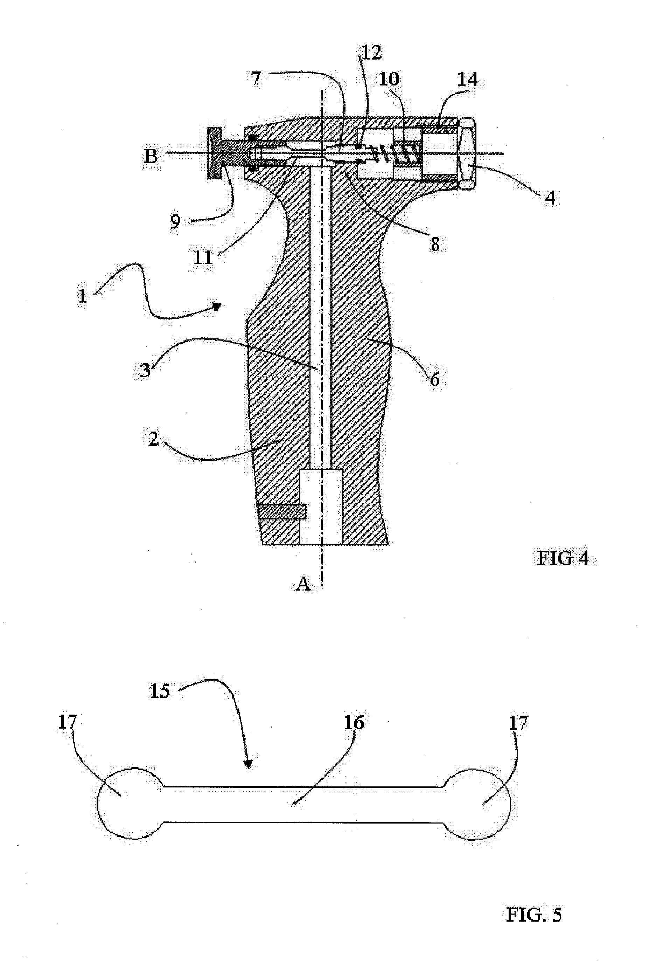

According to the first embodiment of opening 15 (illustrated in figure 5), opening 15 comprises a slit 16 having a width of about 9-10 mm in the direction of said axis (i.e. in the direction defined by the tubular body section 2B in which it is situated) and a transverse width (with respect to said axis) of about 1 mm. Opening 15 also defines at least one widening 17 (or swelling); in the preferred embodiment illustrated, opening 15 defines at its ends two widenings 17 (or swellings); these widenings 17 preferably have a circular shape, with a radius of about 1 mm.

With an opening 15 according to the first embodiment, the jet forms said crossing (or restriction zone) at a distance of about 7-10 cm from opening 15 itself.

According to the second embodiment of opening 15 (illustrated in figure 6), opening 15 comprises a slit 16 having a width of about 14 mm in the direction of said axis (i.e. in the direction defined by the tubular body section 2B in which it is situated) and a transverse width (with respect to said axis) of about 1 mm.

With an opening 15 according to the second embodiment, the jet forms said crossing (or restriction zone) at a distance of about 10-13 cm from opening 15 itself.

It should be noted that, by acting on the handle 6, the person using the device has the possibility of locating the jet in the desired position. If the person wants to wash the external anal area, he or she positions the opening 15 at a greater or lesser distance from the sphincter than the distance at which the jet forms the crossing; in this way the jet does not enter the rectum but washes the area surrounding the sphincter. If the person wants to wash the internal anal area, he or she positions the opening 15 at a distance from the sphincter roughly equal to the distance at which the jet forms the crossing; in this way the jet enters the rectum and washes it.

The opening therefore has an elongated form (slit-shaped) along the direction defined by the tubular body section 2B in which it is situated.

Preferably, the surface of the tubular body 2 forming the aperture (i.e. the radial surface flushed by the liquid which flows through the opening of outlet 5) is smooth and forms a constant section for the aperture.

Device 1 furthermore comprises a projecting element 13 joined to one end of the tubular body 2 in proximity to the outlet 5 of the passage 3, and positioned transversely to prevent said end of the tubular body 2 from being inserted into the rectum. This projecting element 13 therefore (preferably disc-shaped) constitutes means of dissuading a person from any (undesirable) insertion of the tubular body 2 into the rectum.

It should be observed that, preferably, the tubular body 2 comprises a first part (corresponding substantially to the handle 6) forming the inlet 4 to the passage 3 and a second part (corresponding substantially to the part of the tubular body 2 subsequent to the handle 6) forming the outlet 5 to the passage 3 (these parts being distinct and separable from each other), and means for connecting one part to another (and disconnecting them).

Preferably, the first part and the second part are shaped so as to form a bayonet connection, according to a substantially known technique.

This allows the easy replacement of the portion of the device which is destined to get dirty in use, without replacing the part comprising the plugging valve.

It is also envisaged (according to a variant embodiment which is not illustrated) that the tubular body 2 could comprise at least one telescopic portion (according to a technique which is in itself known); for example, it is envisaged that the portion 2A of the tubular body 2 could be telescopic.

This, advantageously, allows the overall dimensions of the device 1 to be reduced as necessary, to facilitate transport.

It should also be noted that device 1 comprises a filter 13, inserted into the passage 3 between the inlet 4 and the plug 7 (or rather the plugging station 8) to filter the liquid.

This ensures that the liquid delivered in the form of a jet by device 1 is always clean (and in particular free from solid bodies), thus allowing the inlet 4 to the passage to be connected to any source of pressurised water.

It is also envisaged (according to a variant embodiment which is not illustrated), that the device 1 could comprise a reservoir (not illustrated because it is of a type which is in itself known) capable of containing said liquid (for example water), removably connected to the tubular body 2 to supply the liquid to the inlet 4 to passage 3, and means operatively active on the liquid in the reservoir in order to keep it under pressure.

In practical terms, it is envisaged that the device 1 could comprise a reservoir filled with pressurised water, connectable to the tubular body 2 at the inlet 4 to passage 3.

This, advantageously, allows the device 1 to be used even in the absence of a source of pressurised water, making the device 1 a self-sufficient (stand alone) device.

The device 1 according to the present invention therefore enables several advantages to be achieved.

In fact, the device 1 allows particularly effective and precise delivery of the jet of water for rectal lavage, which is therefore generated in a gradual manner according to the needs of the person, without the latter having to use both hands to hold the device in position and at the same time open and close the plugging valve.

In particular, actuating the manoeuvring member 9, entailing a direct and coaxial action on the plug 7 (without the interposition of mechanisms or levers), defines a metering system for liquids for achieving the maximum constructional simplicity and the best cleaning action possible.

Therefore the device 1 is particularly easy and convenient to use, even for someone who has never used it before; this is essential in order to permit a person to use the device daily for intimate personal hygiene.

Other particular features of the present invention (the telescopic tubular body and the container of pressurised water, connectable to the inlet) allow the device 1 to be used in any situation, and also facilitate transporting it when travelling.

The particular conformation of the handle 6, of the tubular body (particularly the angle of inclination of the end portion 2B) and of the opening (from which the jet of water is discharged) enable a jet to be generated which is particularly effective in cleaning the rectum and at the same time is not annoying. In particular, this conformation ensures both good ergonomics for manual operation (i.e. a wrist angle which is such as not to limit the pressing action of the thumb), and optimal location in the working position for the end part of the tubular body 2, from which the delivered water is discharged (in other words, the orientation of the jet in the direction of the anal orifice is such as to maximise its effective action).

## Claims

1. Device (1) for rectal lavage, comprising:
- a tubular body (2) defining a passage (3) having an inlet (4) connectible to a source of pressurised liquid and an outlet (5) consisting of an opening defined in a lateral wall of the tubular body (2);
- a plug (7) operatively inserted in the passage (3) in a plugging station (8) and axially movable between positions for opening and closing the passage (3);
- actuating means operatively active on the plug (7) to generate a jet of liquid discharged from said opening and direct it towards the anus for lavage of the rectum,
**characterised in that** said actuating means comprise a manoeuvring member (9) slidingly coupled to the tubular body (2) so as to be movable parallelly to the axis of the plug (7), the manoeuvring member (9) being connected to the plug (7) in order to move it.

2. Device according to claim 1, wherein a section of the passage (3) defining the plugging station (8) is disposed in a substantially radial direction in relation to a longitudinal axis (A) defined by the tubular body (2), the plug (7) and the manoeuvring member (9) being rigidly connected and movable along said radial direction.

3. Device according to claim 1 or 2, wherein the actuating means comprise a rod-shaped element (11) movably coupled to a seat defined by the tubular body (2) and having a first portion projecting from the tubular body (2) to define said manoeuvring member (9) and a second portion operatively active on the passage (3) in the plugging station (8) to define said plug (7).

4. Device according to any one of the preceding claims, wherein:
- the plugging station (8) defines a narrowing of the passage (3), in which the plug (7) is suited to fit in order to close the passage off;
- the plug (7) has a variable diameter that is monotone non-decreasing in the direction of insertion of the plug (7) in the narrowing.

5. Device according to claim 4, wherein the plug (7) comprises a first portion with a diameter increasing up to a prefixed value and a second cylindrical portion with a diameter equal to said prefixed value, situated after the first portion in the direction of insertion of the plug (7) in the narrowing.

6. Device according to any one of the preceding claims, wherein the tubular body (2) is shaped so as to define at least a first portion (2A) and a second portion (2B), which is bent relative to the first at a prefixed angle, said portions being substantially rectilinear.

7. Device according to any one of the claims 1-5, wherein the tubular body (2) is shaped so as to define a first portion (2A) and a second portion (2B), which is bent relative to the first at a prefixed angle, the first portion (2A) defining a first section curved in a direction opposite to that of the second portion (2B).

8. Device according to any one of the preceding claims, wherein the tubular body (2) is shaped so as to define a hand grip (6) at a seat in which the manoeuvring member (9) is coupled, configured so as to enable said manoeuvring member (9) to be actuated with the thumb of the hand that holds the device (1).

9. Device according to any one of the preceding claims, comprising a projecting element (13) joined at one end of the tubular body (2) opposite the inlet (4) of the passage (3) and disposed transversely to prevent said end of the tubular body (2) from being inserted into the rectum.

10. Device according to any one of the preceding claims, wherein the tubular body (2) comprises at least one telescopic portion.

11. Device according to any one of the preceding claims, comprising a reservoir suitable for containing said liquid, removably associated with the tubular body (2) in order to supply liquid to the inlet (4) of the passage (3), and means operatively active on the liquid in the reservoir to keep it under pressure.

12. Device according to any one of the preceding claims, wherein the opening (15) has a shape that is elongated in the direction defined by a portion of the tubular body (2B) in which it is defined.

13. Device according to claim 12, wherein the opening (15) has a cross section of approximately 14 square mm.

14. Device according to claim 12 or 13, wherein the opening comprises a slit (16) extending approximately 14 mm in the direction defined by the portion of the tubular body (2B) and approximately 1 mm in the direction transverse thereto.

15. Device according to claim 12 or 13, wherein the opening comprises a slit (16) extending approximately 9-10 mm in the direction defined by the portion of the tubular body (2B) and approximately 1 mm in the direction transverse thereto, and defines at least one enlarged portion (17).
